# EUROPEAN PATENT APPLICATION

(11) **EP 4 299 565 A1**
(43) Date of publication of application: **03.01.2024**
(21) Application number: 21927635.9
(22) Date of filing: 22.11.2021
(51) Int. Cl.: C07D 277/04, A61K 31/426, A61P 31/04, A61P 1/00

(54) **NOVEL NITROTHIAZOLE DERIVATIVE AND APPLICATION THEREOF**

(30) Priority: 24.02.2021 CN 202110206749
(71) Applicant: Chengdu Biobel Biotechnology Co., Ltd., Sichuan 610093 (CN)
(72) Inventor: GUANG, Bing, Chengdu, Sichuan 610093 (CN); YANG, Tai, Chengdu, Sichuan 610093 (CN); DONG, Renhan, Chengdu, Sichuan 610093 (CN); LIU, Jin, Chengdu, Sichuan 610093 (CN); ZHAN, Wei, Chengdu, Sichuan 610093 (CN); QIN, Chuanjun, Chengdu, Sichuan 610093 (CN); XIE, Jian, Chengdu, Sichuan 610093 (CN); HUANG, Sheng, Chengdu, Sichuan 610093 (CN); PENG, Xiangyang, Chengdu, Sichuan 610093 (CN); LAI, Yongxin, Chengdu, Sichuan 610093 (CN); XU, Qing, Chengdu, Sichuan 610093 (CN); PENG, Jian, Chengdu, Sichuan 610093 (CN)
(74) Representative: Altmann Stößel Dick Patentanwälte PartG mbB
(86) International application number: PCT/CN2021/132174
(87) International publication number: WO 2022/179211

(57) **Abstract**

Provided is a compound represented by formula (I). The compound can effectively inhibit the growth of clostridium difficile; and compared with nitazoxanide and other antibiotics, the compound also has significantly less influence on intestinal probiotics and lower toxic side effects while significantly inhibiting the activity of clostridium difficile, and the disease is not easy to relapse after the drug is stopped. The compound has a very good application prospect in preparation of a drug for preventing and/or treating clostridium difficile infectious diseases, inhibiting the relapse of the clostridium difficile infectious diseases, or treating complications of the clostridium difficile infectious diseases.

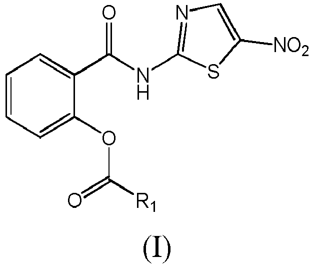

## Description

### Technical field

The present invention belongs to the technical field of medicine, and in particular, relates to a nitazoxanide derivative and the uses thereof.

### Background technology

*Clostridium difficile* is a specialized anaerobic bacterium belonging to the genus *Clostridium,* which is very sensitive to oxygen and difficult to isolate and cultivate. Therefore, it is named *Clostridium difficile* and generally parasitizes in the human intestine. Usually, *Clostridium difficile* infection is caused by excessive use of certain antibiotics, which disrupt the balance of the intestinal microbiota and accelerate the growth rate of the *Clostridium difficile* microbiota, leading to inflammation. *Clostridium difficile* can produce exotoxins A and B, and have different effects in different stages. Toxin A is an enterotoxin, which binds with mucosal cells at the initial stage, producing primary damage, which can cause inflammation, cell invasion, increased permeability of intestinal wall, bleeding and necrosis. Toxin B is a cytotoxin that damages the cytoskeleton, causing cell pyknosis and necrosis, and directly impairs the intestinal parietal cells, leading to diarrhea.

*Clostridium difficile* infectious diseases is a disease caused by the infection of *Clostridium difficile* bacteria and/or *Clostridium difficile* spores. Pseudomembranous enteritis is a common infectious diseases caused by *Clostridium difficile.* Its clinical manifestations are diarrhea and abdominal pain, with systemic toxic symptoms. The symptoms suddenly begin, and are accompanied by low blood pressure, as well as usually accompanied by fever, increased white blood cells, and even death. In addition, the infection of *Clostridium difficile* bacteria and/or *Clostridium difficile* spores may also cause complications of *Clostridium difficile* infectious diseases. Common complications include pyelonephritis, meningitis, abdominal and vaginal infections, bacteremia, gas gangrene, and the same. In recent years, *Clostridium difficile* has become an important pathogen causing infectious diseases in hospitals, and has been paid more and more attention.

Nitazoxanide 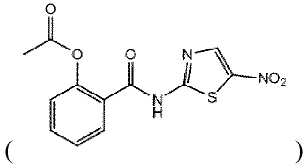 is a derivative of nitrothiazolylsalicylamide, and although its actual mechanism of action is not clear, it is considered to be related to the inhibition of enzyme-dependent electron transfer reaction of pyruvate and ferredoxin oxidoreductase (PFO), which is important for anaerobic energy metabolism. In addition to sporozoans and Giardia intestinalis, nitazoxanide is also effective on many intestinal parasites, such as *Isospora belli*, amoebae, *Ascaris lumbricoides,* hookworms, *Trichuris trichiura*, beef tapeworm, *Hymenolepis nana* and *Fasciola hepatica.* It has also been reported that nitazoxanide has a good effect on *Clostridium difficile* enteritis for which metronidazole is ineffective (Musher DM, Logan N, Mehendiratta V, Yang Liu. Metronidazole is ineffective in treating Clostridium difficile enteritis: nitazoxanide is effective in treatment [J]. Chinese Journal of Infection and Chemotherapy, 2008(01): 46).

Many research reports have confirmed that nitazoxanide and its various derivatives have excellent biological activities, and the structures of the derivatives have different effects on biological activities (Han Zhang. Synthesis of nitazoxanide derivatives and their antibacterial quantitative structure-activity relationship [D]. Chinese Academy of Agricultural Sciences, 2012). Therefore, it is of great significance to further explore and synthesize nitazoxanide derivatives with relatively simple structure and better biological activity, which will open up a new field for the research of such compounds.

### Content of the invention

The object of the present invention is to provide a derivative of nitazoxanide and its use in inhibiting *Clostridium difficile.*

The present invention provides a compound represented by formula (I): 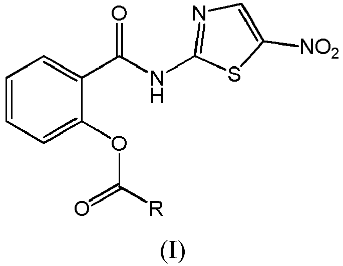 wherein R is selected from the group consisting of C₃₋₁₆ straight or branched alkyls.

Further, the compound is selected from the group consisting of:

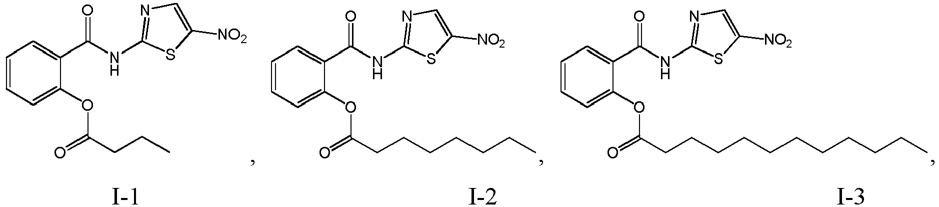

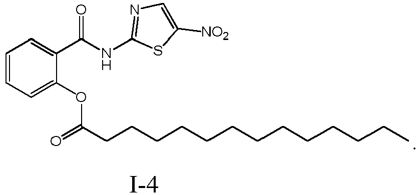

The present invention further provides the use of the compound mentioned above for preparing antibacterial drugs.

Further, the antibacterial agent is a drug which inhibits the growth of *Clostridium difficile.*

Still further, the drug is those for preventing and/or treating *Clostridium difficile* infectious diseases, and/or complications of *Clostridium difficile* infectious diseases, and/or relapse of *Clostridium difficile* infectious diseases.

Further, the drug for preventing and/or treating the complications of *Clostridium difficile* infectious diseases is those for treating and/or preventing the digestive tract infection syndrome caused by *Clostridium difficile* bacterial infection.

Further, the drug for treating and/or preventing the digestive tract infection syndrome is those for treating and/or preventing pseudomembranous enteritis, diverticulitis, antibiotic-related diarrhea, and incomplete or complete intestinal obstruction.

The present invention provides a drug that inhibits the growth of *Clostridium difficile,* which is a composition formed by a compound mentioned above as the active ingredient, with the addition of pharmaceutically acceptable excipients
Further, the pharmaceutically acceptable excipients are selected from one or more of diluents, bulking agents, colorants, glidants, lubricants, adhesives, stabilizers, suspending agents or buffering agents

Even further, the preparation is oral; preferably, the oral preparation is selected from the group consisting of granules, capsules, tablets, pills, suspensions, and emulsions.

The experimental results have shown that compared with nitazoxanide and other antibiotics, the compound of the present invention also has significantly less effect on intestinal probiotics and lower toxic side effects, while significantly inhibits the activity of *Clostridium difficile,* and the disease is not easy to relapse after the drug is stopped. The compound has a very good application prospect in preparation of a drug for preventing and/or treating *Clostridium difficile* infectious diseases, inhibiting the relapse of the *Clostridium difficile* infectious diseases, or treating complications of the *Clostridium difficile* infectious diseases.

Obviously, based on the above content of the present invention, according to the common technical knowledge and the conventional means in the field, without department from the above basic technical spirits, other various modifications, alternations, or changes can further be made. By the following specific examples of said embodiments, the above content of the present invention is further illustrated. But it should not be construed that the scope of the above subject matter of the present invention is limited to the following examples. The techniques realized based on the above content of the present invention are all within the scope of the present invention.

### Description of figures

Figure 1. The body weight percentage curve of golden hamsters in each group of Experimental example 3.
Figure 2. The survival curve of golden hamsters in each group of Experimental example 3.

### Examples

Tizoxanide and other chemical reagents used in the examples of the present invention were purchased from Chengdu Kelong Chemical Reagent Factory.

### Example 1: Synthesis of compound I-1 of the present invention

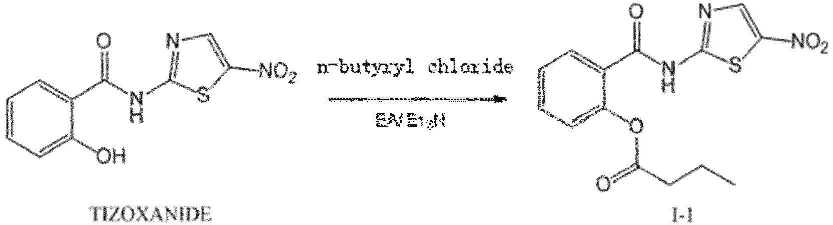

2.0 g of tizoxanide (7.5 mmol) was placed into a 50 ml single-neck bottle, to which were added 20 ml of ethyl acetate and 0.9 g of triethylamine (8.8 mmol), and then 0.9 g of n-butyryl chloride (8.4 mmol) was added dropwise under stirring. After drop addition, the reaction mixture was heated and refluxed for 2 h in an oil bath. After the raw materials could not be detected by TLC, the reaction mixture was lowered to room temperature, and then filtered, to remove the solid. The filtrate was diluted with 20 ml of ethyl acetate, and then washed twice with 10 ml of 0.1 M dilute hydrochloric acid, washed once with 10 ml of 10% sodium bicarbonate solution, and finally washed with 10 ml of saturated NaCl aqueous solution until neutral. The ethyl acetate layer was dried over 2.5 g of anhydrous sodium sulfate for 30 min, and then filtered to remove sodium sulfate. The filtrate was concentrated under reduced pressure until dry, and the resultant crude product was purified by silica gel column chromatography, to obtain compound **I-1**.
¹H NMR (400 MHz, CDCl₃) *δ*: 11.03 (brs, 1H), 8.11-8.05 (m, 1H), 8.02 (d, *J* = 7.9 Hz, 1H), 7.71 (td, *J* = 7.9, 1.7 Hz, 1H), 7.46(t, *J* = 7.6 Hz, 1H), 7.32 (d, *J* = 8.2 Hz, 1H), 2.69 (t, *J* = 7.4 Hz, 2H), 1.90-1.78 (m, 2H), 1.06 (t, *J* = 7.4 Hz, 3H). ESI-MS *m*/*z:* 334.02 [M-1]⁻.

### Example 2: Synthesis of compound I-2 of the present invention

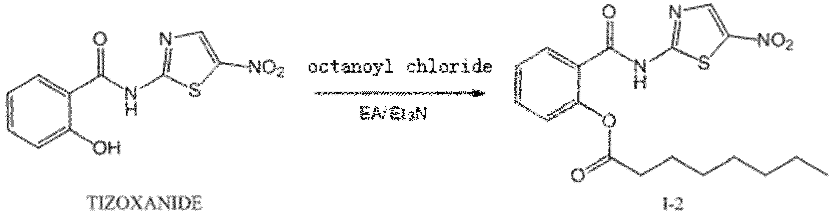

By the synthesis method analogous to Example 1, tizoxanide and octanoyl chloride were used as raw materials, to obtain compound **I-2.**

¹H NMR (400 MHz, CDCl₃) *δ*: 10.86 (br s, 1H), 8.14 (s, 1H), 8.04 (dd, *J* = 7.8, 1.7 Hz, 1H), 7.75-7.66 (m, 1H), 7.46 (t, *J* = 7.6 Hz, 1H), 7.31 (d, *J* = 8.2 Hz, 1H), 2.71 (t, *J* = 7.5 Hz, 2H), 1.85-1.73 (m, 2H), 1.48-1.22 (m, 8H), 0.90 (t, *J* = 6.8 Hz, 3H).

ESI-MS *m*/*z:* 390.06 [M-1]⁻.

### Example 3: Synthesis of compound I-3 of the present invention

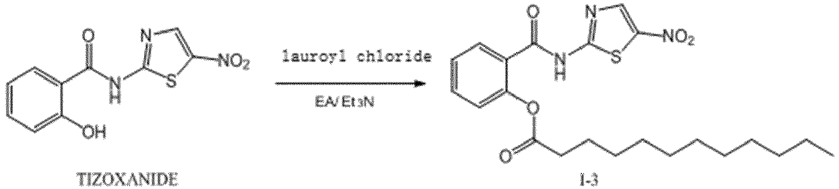

By the synthesis method analogous to Example 1, tizoxanide and lauroyl chloride were used as starting materials, to obtain compound **I-3.**

¹HNMR (400 MHz, DMSO-d₆) *δ*: 13.64 (brs, 1H), 8.70 (s, 1H), 7.82 (d, *J* = 7.7 Hz, 1H), 7.68 (t, *J* = 7.8 Hz, 1H), 7.44 (t, *J* = 7.5 Hz, 1H), 7.30 (d, *J* = 8.2 Hz, 1H), 2.55 (t, *J* = 7.1 Hz, 2H), 1.62-1.43 (m, 4H), 1.32-1.16 (m, 14H), 0.85 (t, *J* = 6.7 Hz, 3H).

ESI-MS *m*/*z:* 446.10 [M-1]⁻.

### Example 4: Synthesis of compound I-4 of the present invention

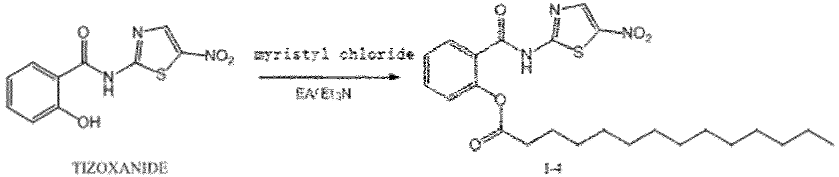

By the synthesis method analogous to Example 1, tizoxanide and myristyl chloride were used as starting materials, to obtain compound **I-4**.

¹HNMR (400 MHz, CDCl₃) *δ*: 10.63 (br s, 1s), 8.26-8.20 (m, 1H), 8.08 (d, *J* = 7.8 Hz, 1H), 7.70 (t, *J* = 7.9 Hz, 1H), 7.47 (t, *J* = 7.5 Hz, 1H), 7.31 (d, *J* = 8.2 Hz, 1H), 2.72 (t, *J* = 7.5 Hz, 2H), 1.86-1.74 (m, 2H), 1.47-1.38 (m, 2H), 1.33-1.25 (m, 18H), 0.90 (t, *J* = 6.7 Hz, 3H). ESI-MS *m*/*z:* 474.15 [M-1]⁻.

### Example 5: Preparation of medicinal tablet composition containing the compound of the present invention

The medicinal tablet composition of compound **I-1** contained 1 part by weight of compound **I-1**, 0.1-0.3 parts by weight of lactose, 0.4-0.2 parts by weight of starch, 0.008-0.014 parts by weight of sodium carboxymethyl starch, an appropriate amount of povidone K30, 0.01-0.05 parts by weight of magnesium stearate, and 0.5 parts by weight of 40% ethanol. The tablets were prepared according to the above ratio, to obtain medicinal tablets of compound **I-1** according to the present invention, with each tablet containing 50-1500 mg of compound **I-1**.

Using the same method as above, medicinal tablet compositions of compounds **I-2**, **I-3**, **I-4**, and **I-5** were prepared, respectively.

### Example 6: Preparation of medicinal capsule composition containing the compound of the present invention

The medicinal capsule composition of compound **I-2** contained 300 g of compound **I-2**, 193 g of microcrystalline cellulose, and 7 g of powdered silica gel, with a total weight of 500 g, which were added to No. 2 vacant capsules; alternatively, the composition may contain 1200 g of compound **I-2**, 279 g of microcrystalline cellulose, and 21 g of powdered silica gel, with a total weight of 1500 g, which were added to No. 00 vacant capsules. The preparation method was as follows:
a) Compound **I-2**, microcrystalline cellulose, and powdered silica gel were mixed, to obtain a mixed powder;
b) After passing through a 120 mesh sieve, the mixed powder was filled into capsules and then sealed, to prepare a total of 1000 capsules.
Each capsule contained 300 mg or 1200 mg of compound **I-2**.

Using the same method as above, medicinal capsule compositions of compounds **I-1**, **I-3**, **I-4**, and **I-5** were prepared, respectively.

### Example 7: Preparation of medicinal suspension composition containing the compound of the present invention

The medicinal suspension composition of compound **I-3** contained 30 g of compound **I-3**, 50 g of gum acacia, 2 g of sodium carboxymethyl cellulose, 2 g of sodium benzoate, 3 g of citric acid, 3 g of sodium citrate, 20 g of sucrose, and 890 g of water. The preparation method was as follows:
a) The gum acacia and sodium carboxymethyl cellulose were passed through a 100 mesh sieve, and then mixed with water, to prepare an adhesive solution, which was placed in an emulsifier;
b) Sodium benzoate, citric acid, sodium citrate, compound **I-3**, and sucrose, that were passed through a 100 mesh sieve, were added in sequence, and then sheared, to obtain the medicinal suspension composition.

Using the same method as above, medicinal suspension compositions of compounds **I-1**, **I-2**, **I-4**, and **I-5** were prepared, respectively.

The beneficial effects of the compound of the present invention were demonstrated by Experimental examples.

### Experimental example 1: In vitro inhibitory activity of the compound according to the present invention on Clostridium difficile

### (1) Experimental method

Three *Clostridium difficile* strains ATCC BBA1870, ATCC 700057, and ATCC 630 were selected for the experiment, and the test was performed using Brucella agar medium with supplements. The strains frozen in glycerol at -80 °C were seeded in solid agar medium. The medium was placed in a 37 °C incubator for anaerobic cultivation for 24-48 h. The testing concentrations of the compound according to the present invention were 32 µg/ml∼0.0156 µg/ml, including a total of 11 concentration gradients obtained by dilution in a ratio of 1:2; nitazoxanide was used as the control compound, while vancomycin was used as the positive control drug. The test concentrations of control compound nitazoxanide and positive control drug vancomycin were 32 µg/ml∼0.0156 µg/ml, including a total of 11 concentration gradients obtained by dilution in a ratio of 1:2 for all control compounds. For each compound of the present invention, a high concentration (100× test concentration) of working solution was prepared and used on the same day, and 100% DMSO was used as the solvent. As for the preparation of each agar plate for each diluted concentration, 20 µl of high concentration working solution was mixed with 2 ml of melted brucella agar with supplements, and then added to a 6-well plate for solidification. 1% DMSO was used as growth control. On the day of the experiment, an appropriate amount of single bacterial colony was selected and suspended in physiological saline. A turbidity meter was used to adjust the turbidity of the bacterial solution to OD₆₀₀ = 0.2, and thus the density was about 1 × 10⁸ CFU/ml. 2 µl of such bacterial solution was directly seeded on a solid dilution agar plate of each compound. Therefore, each well of 6-well plate contained about 10⁵ CFU of *Clostridium difficile,* which was the test plate. Then, all of the test plates were subjected to anaerobic cultivation at 35 ± 2 °C for 48 h. After 48 h cultivation, the minimum drug concentration that could completely or significantly inhibit bacterial growth by observation with naked eyes was the MIC.

### (2) Experimental results

The experimental results are listed in Table 1 below. The results showed that, compared with the control compound nitazoxanide, the compound of the present invention had significantly improved inhibitory effect on three *Clostridium difficile* strains. The compounds of the present invention could effectively inhibit the growth of *Clostridium difficile* bacteria, with a MIC value of ≤ 1 µg/ml for three kinds of *Clostridium difficile* bacteria, indicating significant antibacterial activity. The MIC values against bacterial strains ATCC BBA1870 and ATCC 630 were significantly lower than those of nitazoxanide and vancomycin, suggesting that the inhibitory activity of the compound according to the present invention against *Clostridium difficile* was significantly better than those of nitazoxanide and vancomycin.

**Table 1. The MIC values (µg/ml) for the inhibition of the compound according to the present invention on Clostridium difficile bacteria.**

| Compounds | *Clostridium difficile* | | |
|---|---|---|---|
| | ATCC BBA1870 | ATCC 700057 | ATCC 630 |
| **I-1** | 0.25 | 0.25 | 0.0625 |
| **I-2** | 0.25 | 0.25 | 0.0625 |
| **I-3** | 0.125 | 0.0625 | 0.0625 |
| **I-4** | 0.25 | 0.125 | 0.0625 |
| nitazoxanide | 0.5 | 0.125 | 0.125 |
| vancomycin | 1 | 1 | 2 |

### Experiment example 2: Selectivity of compound I-3 according to the present invention on intestinal probiotics

### (1) Experimental method

Based on the results of Experiment example 1, compound **I-3** of the present invention with the best inhibitory effect on various *Clostridium difficile* strains was selected to verify the selectivity on intestinal probiotics. In the experiment, seven probiotic strains were selected, including *Bifidobacterium bifidum* CGMCC NO.1.5091, *Enterococcusfaecalis* ATCC 19433, ATCC BAA-835, *Lactobacillus acidophilus* ATCC4356, *Lactococcus lactis* subsp. ATCC19435, *Lactobacillus rhamnosus* ATCC7469, *Lactobacillus bulgaricus* ATCC 11842, and the test was performed using Brucella agar medium. The strains frozen at -80 °C were seeded in solid agar medium. The medium was placed in a 37 °C incubator and anaerobically cultivated for 24-48 h. The test concentrations of compound **I-3** were 256 µg/ml∼0.25 µg/ml, including a total of 10 concentration gradients obtained by dilution in a ratio of 1:2; nitazoxanide, vancomycin and fidaxomicin were used as control compounds. The test concentrations of three control compounds were 128 µg/ml∼0.0125 µg/ml, including a total of 10 concentration gradients obtained by dilution in a ratio of 1:2 for all control compounds. For compound **I-3** of the present invention, a high concentration (100× test concentration) of working solution was prepared and used on the same day, and 100% DMSO was used as the solvent. As for the preparation of each agar plate for each diluted concentration, 20 µl of high concentration working solution was mixed with 2 ml of melted brucella agar with supplements (45-55°C), and then added to a 6-well plate for solidification. 1% DMSO was used as growth control. On the day of the experiment, an appropriate amount of single bacterial colony was selected and suspended in physiological saline. A turbidity meter was used to adjust the turbidity of the bacterial solution to OD₆₀₀ = 0.2, and thus the density was about 1 × 10⁸ CFU/ml. 2 µl of such bacterial solution was directly seeded on a solid dilution agar plate of each compound. Therefore, each well of 6-well plate contained about 10⁵ CFU of *Clostridium difficile,* that was the test plate. Then, all of the test plates were subjected to anaerobic cultivation at 35 ± 2 °C for 48 h. After 48 h cultivation, the minimum drug concentration that could completely or significantly inhibit bacterial growth by observation with naked eyes was the MIC.

### (2) Experimental results

The experimental results are shown in Table 2 below. The results suggested that at the same concentration, compared with the control compound nitazoxanide, vancomycin and fidaxomicin, compound **I-3** of the present invention had no significant inhibitory effect on 7 kinds of intestinal probiotic bacteria. Compared with the MIC of inhibiting the growth of *Clostridium difficile* in Experiment example 1, the maximum test concentration of compound **I-3** according to the present invention was much higher than its MIC value against *Clostridium difficile,* indicating that compound **I-3** selectively inhibited the growth of *Clostridium difficile* and had significant selectivity. That is to say, although the antibiotics such as nitazoxanide and vancomycin could inhibit *Clostridium difficile,* they also affect the activity of intestinal probiotics. However, the compound of the present invention not only could effectively inhibit *Clostridium difficile,* but also did not influence the activity of intestinal probiotics, and so the compound had significant advantages.

**Table 2. The MIC value (µg/ml) for compound of the present invention to inhibit the activity of Clostridium difficile.**

| Strains | MIC value | | | |
|---|---|---|---|---|
| | 1-3 | Nitazoxanide | Vancomycin | Fidaxomicin |
| *Bifidobacterium bifidum* CGMCC NO.1.5091 | 128 | 32 | 4 | 4 |
| *Enterococcus faecalis* ATCC 19433 | 256 | 64 | 4 | 1 |
| ATCC BAA-835 | 128 | 1 | 8 | 1 |
| *Lactobacillus acidophilus* ATCC4356 | >256 | 64 | 4 | 128 |
| *Lactococcus lactis* subsp. ATCC1943 5 | 256 | 8 | 4 | 64 |
| *Lactobacillus rhamnosus* ATCC7469 | >256 | 256 | 256 | 16 |
| *Lactobacillus bulgaricus* ATCC11842 | >256 | 64 | >256 | 128 |

### Experimental example 3: Treatment of Clostridium difficile infection in golden hamsters with compounds of the present invention

### (1) Experimental method

60 of 5-week-old male golden hamsters, weighing 70-90 g, were purchased from Beijing Vital River Laboratory Animal Technology Co., Ltd.. After one week of adaptive feeding, golden hamsters were randomly divided into six groups. The model group and four test groups were orally administrated 10 mg/kg clindamycin, while the blank group was given the same volume of normal saline, and this day was recorded as day -1. On day 0, the model group and the test groups were challenged with *Clostridium difficile* spores (ATCC 630 strain), and each hamster received 5E4 spores. The blank group was not treated. Starting from the first day, four test groups were administered 100 mg/kg of compound **I-3** according to the present invention, 10 mg/kg of vancomycin, 10 mg/kg of fidaxomicin, and 100 mg/kg of nitazoxanide twice a day, respectively; the blank group and the model group were given equal amounts of normal saline for a total of 20 days. During this period, the changes in weight, the diarrhea, and the mortality of animals were observed. Starting from the 21st day, the drugs were removed, but the observation was continued until the 45th day.

### (2) Experimental results

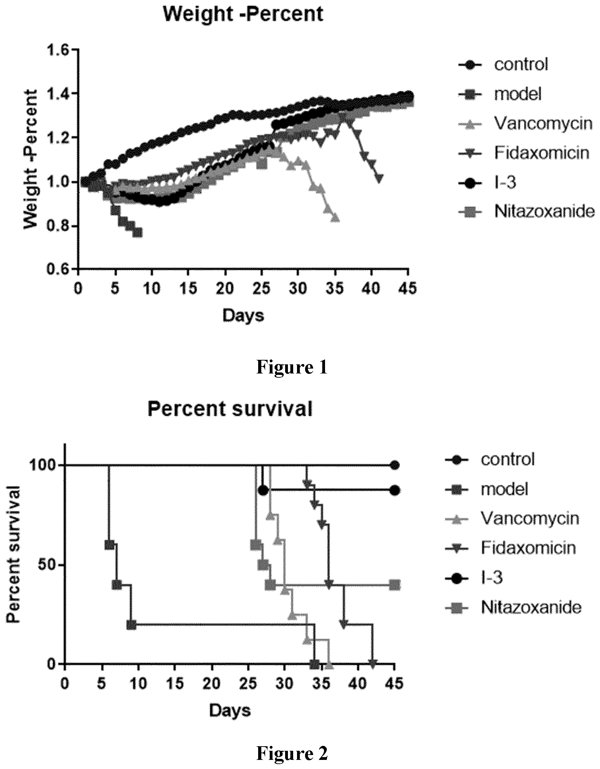

The percent changes in body weight of golden hamsters in each group is recorded in Figure 1, while the survival curve for each group of golden hamsters is recorded in Figure 2. The model group gradually died within 5∼10 days after infection with *Clostridium difficile,* and ultimately all hamsters died. The animal in each test group did not die during the first 20 days of treatment, but 5∼10 days after drug withdrawal, hamsters in the control groups, i.e. the nitazoxanide group and the vancomycin group, gradually experienced deaths, and in the end, four hamsters in the nitronide group survived, while all hamsters in the vancomycin group died. 15 days after withdrawal, the hamsters in fidaxomicin group also began to die one after another, and ultimately, all of them died. In compound **I-3** group, only one animal died 7 days after withdrawal, while all other hamsters survived. Compared with the control compounds vancomycin, fidaxomicin and nitazoxanide, compound **I-3** of the present invention not only inhibited the growth of *Clostridium difficile* during the treatment period, but also could prevent the recurrence caused by *Clostridium difficile* spore germination after drug withdrawal, indicating that the therapeutic effect of the compound according to the present invention on golden hamsters infected with *Clostridium difficile* was significantly better than those of vancomycin, fidaxomicin and nitazoxanide, and thus the compound had a very good application prospect in clinic.

In summary, the compound of the present invention could effectively inhibit the growth of *Clostridium difficile*; and compared with nitazoxanide and other antibiotics, the compound not only significantly suppressed the activity of *Clostridium difficile,* but had significantly less effect on intestinal probiotics and lower toxic side effects; and the disease was not easy to relapse after the drug was stopped. Thus, the compound had a very good application prospect in preparation of a drug for preventing and/or treating *Clostridium difficile* infectious diseases, inhibiting the relapse of the *Clostridium difficile* infectious diseases, or treating complications of the *Clostridium difficile* infectious diseases.

## Claims

1. Compounds represented by formula (I): 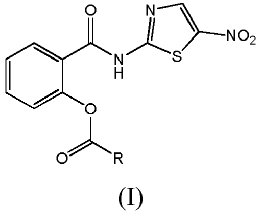 wherein R is selected from the group consisting of C₃₋₁₆ straight or branched alkyls.

2. The compound according to claim 1, **characterized in that** the compound is selected from the group consisting of:

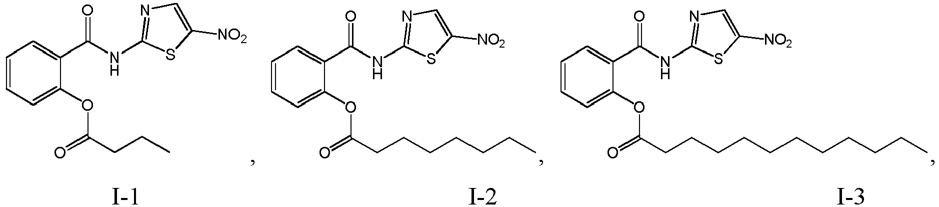

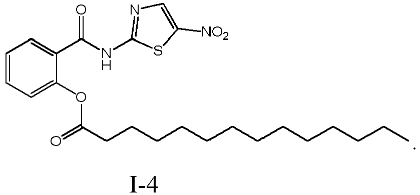

3. Use of the compound according to claim 1 or 2 for preparing antibacterial drugs.

4. The use according to claim 3, **characterized in that** the antibacterial agent is a drug which inhibits the growth of *Clostridium difficile.*

5. The use according to claim 4, **characterized in that** the drug is those for preventing and/or treating *Clostridium difficile* infectious diseases, and/or complications of *Clostridium difficile* infectious diseases, and/or relapse of *Clostridium difficile* infectious diseases.

6. The use according to claim 5, **characterized in that** the drug for preventing and/or treating the complications of *Clostridium difficile* infectious diseases is those for treating and/or preventing the digestive tract infection syndrome caused by *Clostridium difficile* bacterial infection.

7. The use according to claim 6, **characterized in that** the drug for treating and/or preventing the digestive tract infection syndrome is those for treating and/or preventing pseudomembranous enteritis, diverticulitis, antibiotic-related diarrhea, and incomplete or complete intestinal obstruction.

8. A drug that inhibits the growth of *Clostridium difficile,* **characterized in that** it is a composition formed by a compound according to claim 1 or 2 as the active ingredient, with the addition of pharmaceutically acceptable excipients

9. A drug according to claim 8, **characterized in that** the pharmaceutically acceptable excipients are selected from one or more of diluents, bulking agents, colorants, glidants, lubricants, adhesives, stabilizers, suspending agents or buffering agents

10. A drug according to claim 8 or 9, **characterized in that** the preparation is oral; preferably, the oral preparation is selected from the group consisting of granules, capsules, tablets, pills, suspensions, and emulsions.
